**Europäisches Patentamt**

**European Patent Office**

**Office européen des brevets**

(11) Numéro de publication: **0 112 741**
**A1**

## (12) DEMANDE DE BREVET EUROPEEN

(21) Numéro de dépôt: **83402230.3**

(22) Date de dépôt: **18.11.83**

(51) Int. Cl.³: **C 12 N 15/00**, C 12 N 5/00,
C 12 P 21/00, G 01 N 33/54

(30) Priorité: **18.11.82 FR 8219338**

(43) Date de publication de la demande: **04.07.84**
**Bulletin 84/27**

(84) Etats contractants désignés: **AT BE CH DE FR GB IT LI LU NL SE**

(71) Demandeur: **INSTITUT PASTEUR, 25-28, rue du Docteur Roux, F-75724 Paris Cedex 15 (FR)**

(72) Inventeur: **Horaud, Florian, 2 rue Albert de Mun, F-92190 Meudon (FR)**
Inventeur: **Blondel, Bruno, 16 rue Oberkampf, F-75011 Paris (FR)**
Inventeur: **Crainic, Radu, 7 rue Louise Thuliez, F-75019 Paris (FR)**

(74) Mandataire: **Gutmann, Ernest et al, Cabinet Plasseraud 84, rue d'Amsterdam, F-75009 Paris (FR)**

(54) **Anticorps monoclonaux neutralisants à l'égard des structures peptidiques du poliovirus responsables de l'induction d'une immunité antipoliomyélitique; hybridomes secréteurs de tels anticorps et procédé pour leur production.**

(57) L'invention concerné des antícorps monoclonaux neutralisants à l'égard de structures peptidiques du poliovirus responsables de l'induction d'une immunité antipoliomyélitique ainsi que les hybridomes sécréteurs correspondants. Ceux-ci sont formés par fusion de cellules spléniques d'un animal prélablement immunisé contre le poliovirus correspondant à antigénicité «C» et de cellules myélomateuses appropriées, puis par sélection de ceux des hybridomes sécréteurs d'anticorps actifs à la fois contre les poliovirus à antigénicité «C» et «D» et contre le peptide structural de capside VP1.

EP 0 112 741 A1

1

Anticorps monoclonaux neutralisants à l'égard des structures peptidiques du poliovirus responsables de l'induction d'une immunité antipoliomyélitique ; hybridomes sécréteurs de tels anticorps et procédé pour leur production.

L'invention concerne des anticorps monoclonaux à l'égard de ceux des polypeptides structuraux du poliovirus, qui jouent un rôle dans la séroneutralisation virale, donc des structures du virus responsables de l'induction de l'immunité antipoliomyélitique. Elle concerne également les hybridomes capables de produire de tels anticorps et un procédé pour leur obtention.

On sait qu'il existe trois types sérologiques de poliovirus (types 1, 2 et 3) qui ont les mêmes caractères biologiques et la même propriété de provoquer chez l'homme la poliomyélite paralytique. Les trois types sont séparables les uns des autres en faisant agir les anticorps dans un test de reconnaissance sérologique. En effet, l'anticorps neutralise spécifiquement la propriété du poliovirus de se répliquer dans la cellule animale.

Quel que soit le type auquel il appartient, le virus infectieux porte une antigénicité caractéristique, définie comme l'état antigénique "D". Lorsqu'il est soumis à certains traitements physiques ou chimiques (chauffage à 56°C, ultraviolets, pH élevé, traitement par le phénol), le virus perd son antigénicité "D" et acquiert une nouvelle antigénicité appelée "C". C'est l'antigénicité "D" qui paraît responsable de la formation des anticorps neutralisants. (BLONDEL B. et CRAINIC R. (1981), Relations entre la structure et les propriétés antigéniques des picornavirus. Bull. Inst. Pasteur, 79, 79-97).

Il sera également fait référence dans ce qui suit à des particules "C" et "D" pour désigner les poliovirus présentant ces antigénicités "C" et "D" respectivement.

En dépit de l'existence de vaccins antipolio-

myélitiques efficaces (vaccins à virus tué, vaccins oraux à virus atténué), la situation de la poliomyélite dans le monde reste très inquiétante. Les statistiques de l'OMS ont enregistré en 1980 42.000 cas paralytiques dans 136 pays (OMS (1981). Relevé Epidémiol. Hebd., 56, 329).

Pour satisfaire les besoins en vaccins antipoliomyélitiques pour le programme élargi de vaccinations de l'OMS, et pour combattre efficacement la poliomyélite, de grandes quantités de vaccins, à bon marché, seront nécessaires. Une solution de l'avenir devrait être trouvée dans des vaccins dans lesquels le virus entier inactivé ou atténué serait remplacé par une ou plusieurs "sous-unités" virales ou polypeptides purifiés ou obtenus par synthèse, notamment par la mise en oeuvre des techniques du génie génétique. Ces polypeptides de synthèse correspondraient à ceux des polypeptides structuraux qui jouent un rôle essentiel dans l'induction de l'immunité antipoliomyélitique. De tels vaccins sous-unités ne poseront aucun risque potentiel et le contrôle et la caractérisation seront effectués par des méthodes immunologiques et biochimiques in vitro.

L'invention a pour but la production de moyens, plus particulièrement d'anticorps monoclonaux aisément accessibles, qui permettent la reconnaissance sélective de celles des structures peptidiques qui seraient à leur tour capables d'induire in vivo des anticorps neutralisants à l'égard du virus infectieux, par conséquent de virus porteurs de l'antigénicité "D".

L'invention avait donc pour but la réalisation d'hybridomes capables de permettre la production de tels anticorps monoclonaux, à partir d'un matériau immunologique aisément accessible aux expérimentateurs.

On rappellera au préalable que les anticorps monoclonaux actifs contre un antigène déterminé peuvent être produits par des cellules hybrides (hybridomes) obtenues par fusion de cellules spléniques d'un animal immunisé au préalable contre cet antigène et de cellules de tumeurs myélomateuses. Une telle technique a été décrite pour la première
fois par KOHLER G. et MILSTEIN C. (1975) (Continuous
cultures of fused cells secreting antibody of pre-defined
specificity. Nature, 256, 495).De tels anticorps monoclonaux
sont          dirigés contre un seul déterminant antigénique, et constituent donc un matériel de choix pour l'étude
de la variation antigénique.

Les hybridomes selon l'invention sont caractérisés
en ce qu'ils produisent des anticorps actifs contre à la
fois les particules C et D originaires d'un même poliovirus.

L'invention concerne plus particulièrement encore parmi les hybridomes de ce type ceux qui produisent
des anticorps monoclonaux sélectivement actifs à l'égard
de VP1, l'un des polypeptides de structure de la capside
du poliovirus,.ces anticorps monoclonaux étant par conséquent inactifs à l'égard de VP2 et VP3, les deux autres
polypeptides de structure de la capside du poliovirus.

Cette sélectivité supplémentaire corrobore par
conséquent les constatations qui ont été faites par BLONDEL
et coll., qui, dans une note à l'Académie des Sciences de
Paris (C.R. Acad. Sc. Paris, 294, 11 Janvier 1982. Série
III. 91-94), avaient en effet rapporté que l'induction
d'anticorps neutralisants antipoliovirus type 1 (souche
Mahoney) pouvait être obtenue par l'immunisation du lapin
avec l'un des polypeptides structuraux de la capside du
poliovirus, à savoir VP1, celui-ci ayant préalablement été
isolé du virus  et séparé par électrophorèse en gel de polyacrylamide-dodécylsulfate de sodium et extraction à partir
du gel par électroélution. Par contre, des sérums anti-VP2
et anti-VP3, obtenus par immunisation du lapin avec les
deux autres polypeptides structuraux VP2 et VP3 préparés au
préalable par les mêmes techniques, n'avaient pas montré
d'activité séroneutralisante significative.

Le procédé de formation des hybridomes selon

4

l'invention est caractérisé en ce que l'on réalise une fusion entre des cellules spléniques d'un animal, tel que la souris, préalablement immunisé par un virus ou virion, notamment de type 1, ayant acquis une antigénicité "C" (et ayant par conséquent perdu son antigénicité "D"), et des cellules myélomateuses appropriées, en mettant en oeuvre une technique qui en tant que telle est connue, que l'on cultive lesdites cellules et sélectionne ceux des clones cellulaires qui s'avèrent produire des anticorps monoclonaux actifs non seulement contre lesdits virions à antigénicité "C", mais encore aussi contre les virions homologues à antigénicité "D".

De préférence encore, on sélectionne parmi les hybridomes ainsi isolés ceux d'entre eux qui produisent des anticorps actifs contre VP1.

Conformément à une variante du procédé selon l'invention, on sélectionne dans la dernière étape du procédé ci-dessus ceux des anticorps actifs à la fois contre les virions à antigénicité "C" et contre l'un des peptides structuraux de capside VP1, VP2 ou VP3.

L'invention concerne évidemment encore les anticorps eux-mêmes, ceux-ci étant également définis par leur capacité à neutraliser les différents types d'antigènes susmentionnés.

Le fait d'obtenir des anticorps sélectifs, à l'égard tant du virus infectieux que de VP1, à partir de virions ayant une antigénicité "C", est d'autant plus remarquable, qu'à l'inverse des expériences menées en parallèle, des hybridomes secréteurs d'anticorps monoclonaux actifs contre les virions à antigénicité "C" ou contre VP1 n'ont pu être obtenus par fusion de cellules spléniques d'un animal préalablement immunisé par un virion à activité "D", et des mêmes cellules myélomateuses.

Ce résultat est encore à considérer à la lumière de la publication de J. ICENOGLE et coll. (Virology, (1981), 115, 211-215). Ces auteurs ont en effet indiqué qu'il ne leur avait pas été possible - à partir d'hybridomes résultant de la fusion des cellules myélomateuses qu'ils avaient choisies et de cellules spléniques de souris préalablement immunisées avec un vaccin antipoliovirus purifié de la souche LSC 2ab - d'obtenir des anticorps monoclonaux actifs contre l'un quelconque des polypeptides VP1, VP2, VP3 et VP4 libéré par des virions chauffés pendant 5 minutes au sein d'une solution de dodécyl-sulfate de sodium à 1 %, guère davantage à l'égard de virus "D" chauffés au préalable et ayant acquis une antigénicité "C".

L'invention fournit par conséquent à partir d'un matériau aisément accessible (les particules "C") des anticorps monoclonaux capables de détecter un déterminant antigénique spécifique commun porté à la fois par les particules "C" et "D" du poliovirus, et par VP1, et ce apparemment de façon indépendante de la conformation spatiale des régions peptidiques qui l'entourent. Ce résultat est d'autant plus remarquable que, comme on l'a indiqué plus haut, de tels anticorps monoclonaux n'ont pas été accessibles dans des expérimentations semblables à partir de particules "D".

Ces anticorps monoclonaux constituent donc un réactif de choix pour la détection d'une immunogénicité spécifique, particulièrement utile dans la recherche des sous-unités peptidiques susceptibles d'induire des anticorps neutralisant l'immunogénicité du virus infectieux. D'une façon générale, ces anticorps monoclonaux vont permettre :

a) l'étude des relations générales entre la structure antigénique du poliovirus et ses propriétés antigéniques,

b) l'inventaire et la topographie des différents épitopes viraux et leur caractérisation chimique et immunologique,

c) l'identification et la purification des po-

6

lypeptides produits, par exemple par les moyens du génie
génétique, pour la préparation d'un vaccin poliomyélitique
synthétique.

Les anticorps monoclonaux selon l'invention
peuvent être obtenus à partir de toutes particules virales
ayant acquis une antigénicité "C", de préférence celles
obtenues par un traitement thermique préalable, par exemple
par chauffage pendant 1 heure à 56°C (particules "C").

Les anticorps monoclonaux actifs à la fois contre les particules de poliovirus "C", et sélectivement contre soit VP1, soit VP2, soit VP3, entrent également dans le
champ de l'invention, en tant que réactifs biologiques permettant de reconnaître tout polypeptide ayant les spécificités antigéniques de VP1, VP2 ou VP3. Aucun des anticorps actifs contre VP2 et VP3 ne s'est cependant révélé également
actif contre le virus infectieux. Entrent également dans le
cadre de l'invention les hybridomes sécréteurs de ces anticorps monoclonaux, également obtenus à partir de particules
"C".

Des caractéristiques supplémentaires de l'invention apparaîtront encore au cours de la description d'exemples de fabrication d'hybridomes sécréteurs d'anticorps
monoclonaux répondant aux caractéristiques sus-définies.

1. PREPARATION DES HYBRIDOMES.

1.1. Immunisation des souris.

1.1.1. Antigène. L'antigène pour l'immunisation des animaux
est constitué d'une suspension de poliovirus type 1 (Mahoney)
dénaturé thermiquement (particules C).

Les virus sont d'abord inoculés sur des cultures
de cellules humaines. On a     utilisé les cellules HEp-2 (ATCC CCL23) qui proviennent d'un cancer humain. Lorsque les cellules sont complètement détruites par le virus, on récolte le liquide surnageant qui contient le virus. Cette suspension virale est ensuite ultracentrifugée pendant 3 heures à 100.000 g pour concentrer le virus,et chauffée pendant 1 heure à 56°C.

La suspension finale doit contenir une quantité de virus équivalente à environ $10^9$ unités infectieuses par millilitre.

1.1.2. Schéma d'immunisation.

Des groupes de 3 - 4 souris Balb/c reçoivent par voie intrapéritonéale une première injection de virus (0,2 ml). Après 4 semaines, une deuxième injection avec la même quantité de virus est effectuée, cette fois par voie intraveineuse.

Trois à quatre jours après la dernière injection, les souris sont sacrifiées et la rate est prélevée et mise en culture.

1.2. Mise en culture, fusion et sélection (myélome + cellules de rate).

Les cellules spléniques sont fusionnées avec les cellules HGPRT déficientes de la lignée myélomateuse P3/x63-Ag 8 (BUTTIN G. (1978). Lymphocyte hybridomes. Current Topics in Microbiology and Immunology. 81, Ed. Melchers F., Spring Verlag, Berlin, p. 27). Les mélanges des cellules parentales sont incubés en présence de polyéthylène glycol (PEG) 100 à 30 %. Après élimination du PEG, les cellules sont distribuées dans des plaques à cupules multiples. Après 48 heures d'incubation, le milieu sélectif contenant de l'azaserine (10 µM) et de l'hypoxanthine (50 µM) est ajouté. Ce milieu ne permet la croissance que des seules cellules hybrides, dont les colonies deviennent visibles vers le 8ème jour. A partir du 14ème jour, les surnageants des cupules contenant les colonies hybrides suffisamment développées sont testés pour la présence des anticorps anti- polio (voir § 2). Les hybridomes positifs sont sous-clonés par la technique des dilutions limites pour, d'une part, s'assurer du caractère monoclonal des anticorps sécrétés et, d'autre part, dans certains cas obtenir la stabilité de la sécrétion.

1.3. Sélection des hybridomes sécrétant des anticorps anti-polio.

Tous les clones cellulaires obtenus après la fusion et la sélection ne sécrètent pas les anticorps souhaités. Il est donc nécessaire de sélectionner :

a) les clones cellulaires fabriquant des anticorps, et

b) les clones sécrétant une certaine catégorie d'anticorps.

Dans ce but, on a utilisé la réaction immunoenzymatique appelée aussi ELISA (AVRAMEAS S. et TERNYNCK T. (1971). Peroxidase labelled antibody and Fab conjugates with enhanced intracellular penetration. Immunochem., 8, 1175. VOLLER A., BIDWELL D. et BARTLETT A. (1976). Microplate enzyme immunodiagnosis of virus infection. in : Manual of clinical Immunology. Ed. Rose N.R. & Friedman H. Amer. Soc. Microbiol. p. 506).

2. DETECTION DES ANTICORPS MONOCLONAUX.

Test immunoenzymatique.

L'antigène de référence (le virus utilisé comme antigène immunisant) est fixé sur un support solide (ex. : plaques à 96 cupules en polystyrène, membranes de nitrate de cellulose). Les surnageants des cultures d'hybridomes sont mis en contact avec les surfaces recouvertes d'antigène. Si des anticorps monoclonaux (AM) homologues à l'antigène existent, ils vont être retenus par l'antigène. Leur présence peut être ensuite détectée avec des anticorps anti-souris marqués à la péroxydase. La présence de la péroxydase détermine le changement de couleur d'un réactif qui contient le substrat spécifique de l'enzyme ($H_2O_2$). Donc, tout change-

ment de couleur va indiquer la présence dans le surnageant respectif des AM reconnaissant l'antigène utilisé dans le test.

3. PREPARATION DES ANTICORPS MONOCLONAUX.

3.1. Surnageant de culture d'hybridome.

Les cellules d'hybridome sont mises à pousser en culture in vitro dans du milieu Eagle modifié par Dulbecco, additionné de 10 % de sérum foetal de veau. Le liquide surnageant contenant les anticorps monoclonaux sécrétés par les hybridomes est récolté quand la densité cellulaire dépasse $1 \times 10^6$ cellules par ml et débarrassé des cellules par centrifugation.

3.2. Liquide d'ascite de souris.

Des souris Balb/c sont inoculées par voie intrapéritonéale avec $1 \times 10^6 - 1 \times 10^6$ cellules d'hybridome, après avoir été sensibilisées par la même voie avec du pristane 1 à 5 semaines avant. Le liquide d'ascite contenant les anticorps monoclonaux est prélevé par ponction intrapéritonéale 10 - 14 jours après l'inoculation des cellules. Il est ensuite clarifié par centrifugation.

Le liquide d'ascite contient des anticorps monoclonaux à une concentration environ 100 fois plus élevée que le surnageant de culture correspondant.

4. CARACTERISATION DES ANTICORPS MONOCLONAUX.

4.1. Immunoprécipitation.

La spécificité des anticorps monoclonaux dans le liquide d'ascite vis-à-vis des polypeptides structuraux (VP1, VP2 et VP3) du poliovirus homologue (le même que celui utilisé pour l'immunisation des souris) a été déterminée par le test d'immunoprécipitation (KESSLER S.W. (1975). Rapid isolation of antigens with a staphylococcal protein A antibody absorbent. J. Immunol., 115, 1617-1624). Le même test a été utilisé pour la détection des motifs antigéniques reconnaissables par les anticorps monoclonaux sur les particules C et D du poliovirus homologue.

4.1.1. Immunoprécipitation avec des polypeptides structuraux.

Le poliovirus marqué à la méthionine $^{35}$S (CRAI-NIC R., HORODNICEANU F. et BARME M. (1980) - Quantitation of cell DNA in the evaluation of heteroploid cell as substrate for the preparation of killed viral vaccine. Intervirology, 14, 300-309) a été purifié par centrifugation isopycnique dans un gradient de CsCl (particules D). Les particules C ont été préparées par traitement thermique des particules D à la température de 56°C. Les polypeptides structuraux ont été séparés par électrophorèse en gel de polyacrylamide (12,5 %) en présence de SDS (EGPA-SDS) (LAEMMLI U.K. (1970). Cleavage of structural proteins during the assembly of head bacteriophage T4. Nature (London), 227, 680-685). Les bandes correspondant aux polypeptides structuraux VP1, VP2 et VP3 ont été localisées en colorant un couloir de chaque côté du gel, puis découpées dans la partie du gel non colorée.

Les polypeptides structuraux VP1, VP2 et VP3 marqués à la méthionine $^{35}$S ont ensuite été électroélués du gel dans du tampon 4 mM Tris-base, 2 mM Na acétate, 2 mM EDTA, pH 8,4, en utilisant un appareil ISCO. Des prélèvements (15 µl) de chaque préparation protéique contenant environ 8000 cpm ont été dilués dans 0,5 ml de tampon 150 mM NaCl, 5 mM EDTA, 50 mM Tris, pH 7,4 et 0,02 % NaN$_3$ (tampon NET) contenant 0,05 % du détergent connu sous la désignation "Nonidet P-40" (NP40) et incubés pendant 2 heures à la température de la pièce avec 25 µl d'ascite. Les immunocomplexes ont ensuite été précipités en utilisant des bactéries Staphylococcus aureus et analysés par EGPA-SDS.

L'autoradiographie des gels a montré la spécificité des anticorps monoclonaux contenus dans les ascites testées.

4.1.2. Immunoprécipitation des ascites avec les particules
D et C.

Les ascites ont également été testées vis-à-vis des particules D et C du poliovirus marquées à la méthionine $^{35}$S. Les particules C ont été obtenues en incubant les particules D purifiées par centrifugation isopycnique en gradient de CsCl pendant 1 heure à 56°C.

Des prélèvements de 15 µl de chaque préparation contenant 25.000 cpm ont été ajoutés respectivement à 0,5 ml de tampon NET contenant 0,05 % de NP40 et incubés avec 25 µl d'ascite. Les immunocomplexes ont été précipités comme décrit précédemment, puis la radioactivité précipitée a été mesurée.

4.2. Neutralisation virale.

Le surnageant de culture d'hybridome, ou le liquide d'ascite à tester, est mis en contact 2 heures à 37°C avec 100 doses cytopathogènes 50 (DCP 50) de poliovirus homologue (le même que celui utilisé pour l'immunisation des souris). L'effet cytopathogène restant est déterminé sur une culture de cellules HEp-2. La concentration des anticorps neutralisants est calculée comme l'inverse de la dilution limite qui inhibe l'effet cytopathogène d'une dose de virus (100 DCP 50).

5. UTILISATION DES ANTICORPS MONOCLONAUX ANTI-VP.

Exemple : Les liquides d'ascite contenant des anticorps monoclonaux anti-VP, classifiés par rapport à leur spécificité de polypeptide, ont été testés pour leur capacité à reconnaître des motifs antigéniques sur les particules C et D du poliovirus homologue (par immunoprécipitation, § 4.1.), ainsi que pour leur pouvoir neutralisant sur la particule infectieuse (par neutralisation virale, § 4.2.).

Le degré d'immunoprécipitation des particules C et D a été représenté dans le tableau 1. Les pourcentages ont été calculés d'après les valeurs de radioactivité précipitée par chaque liquide d'ascite testé (valeurs corrigées

12

pour le bruit de fond correspondant au liquide d'ascite "normal" – ASN –) provenant d'une souris inoculée avec des cellules de myélome non productrices d'anticorps monoclonaux). La capacité neutralisante des anticorps monoclonaux est enregistrée dans la dernière colonne.

### TABLEAU 1

| Liquide d'ascite | Spécificité vis-à-vis des protéines structurales | % immunoprécipitation des particules de poliovirus | | Titre neutralisant |
|---|---|---|---|---|
| | | C | D | |
| AS 2 | VP1 | 87 | 84 | 256 |
| AS 3 | VP1 | 89 | 78 | 362 |
| AS 4 | VP1 | 85 | O | 3 |
| AS 16 | VP1 | 81 | O | 3 |
| AS 18 | VP2 | 74 | O | NT |
| AS 1 | VP3 | 91 | O | 3 |
| AS 8 | VP3 | 94 | O | 3 |
| AS 11 | VP3 | 63 | O | 3 |
| AS 13 | VP3 | 88 | O | NT |
| AS 14 | VP3 | 89 | O | 3 |
| AS N | – | O | O | 3 |

NT = non testé

ASN = ascite provenant d'une souris inoculée avec des cellules de myélome non productrices d'anticorps monoclonaux.

Tous les liquides d'ascite testés contiennent des anticorps monoclonaux qui réagissent avec des particules C du poliovirus. Seuls deux des anticorps monoclonaux anti-VP1 (AS 2 et AS 3) reconnaissent un épitope sur la particule D (particule infectieuse), ce qui est en parfaite concordance

0112741

13

avec leur pouvoir neutralisant.

Les hybridomes sécréteurs des anticorps monoclonaux mis en jeu dans la production de AS 2 et AS 3 ont été déposés à la Collection Nationale des Cultures de Microorganismes de l'Institut Pasteur de Paris (C.N.C.M.) respectivement sous les N° I-208 et N° I-209.

Comme il va de soi, et comme il résulte d'ailleurs déjà de ce qui précède, l'invention ne se limite nullement à ceux de ses modes de réalisation et d'application qui ont été plus spécialement envisagés ; elle en embrasse, au contraire, toutes les variantes. A ce titre, elle concerne en particulier les anticorps monoclonaux sélectifs qui peuvent être obtenus en ayant recours à des procédures semblables à celles qui ont été décrites, à partir de particules "C" de poliovirus de type 2 ou 3.

Il est entendu que toute la littérature technique antérieure, plus particulièrement les publications citées dans le corps de la présente description peuvent être considérées comme faisant partie de celle-ci, en ce qui concerne si besoin l'explicitation ou la définition des parties de l'état de la technique auxquelles il a été fait référence, mais qui ne font pas, en tant que telles, partie de l'invention.

14

REVENDICATIONS

1. Hybridomes caractérisés en ce qu'ils produisent des anticorps monoclonaux actifs contre à la fois les particules "C" et "D" originaires d'un même poliovirus.

2. Hybridomes selon la revendication 1, caractérisés en ce que les anticorps monoclonaux qu'ils sont capables de produire sont également actifs à l'égard du polypeptide structural VP1 de la capside du poliovirus correspondant.

3. Hybridomes selon la revendication 1 ou 2, caractérisés en ce que le poliovirus ou les éléments de structure dérivée de celui-ci, et contre lesquels les susdits anticorps sont actifs, sont du type 1.

4. Procédé de préparation d'un hybridome conforme à l'une quelconque des revendications 1 à 3, caractérisé par la réalisation d'une fusion entre des cellules spléniques d'un animal préalablement immunisé par un virus ou virion présentant une antigénicité "C" et des cellules myélomateuses appropriées, en mettant en oeuvre une technique en soi connue, par la culture desdites cellules et par la sélection desdits clones cellulaires qui s'avèrent produire des anticorps monoclonaux actifs non seulement contre les virions à antigénicité "C", mais également contre les virions homologues à antigénicité "D".

5. Procédé selon la revendication 4, caractérisé en ce que le virus ou virion, contre lequel le susdit animal est préalablement immunisé, est du type 1.

6. Procédé selon la revendication 4 ou 5, caractérisé en ce que l'on sélectionne parmi les hybridomes sélectionnés ceux d'entre eux qui produisent des anticorps en outre actifs contre VP1.

7. Procédé selon la revendication 6, caractérisé en ce que le poliovirus à antigénicité "C" mis en eouvre est un poliovirus du type 1 (Mahoney).

8. Procédé de formation d'hybridomes, caractérisé en ce que l'on réalise une fusion entre des cellules spléniques d'un animal préalablement immunisé par un virus ou virion présentant une antigénicité "C" et des cellules myélomateuses appropriées en mettant en oeuvre une technique en soi connue, l'on cultive lesdites cellules et sélectionne lesdits clones cellulaires qui s'avèrent produire des anticorps monoclonaux actifs non seulement contre les virions à antigénicité "C", mais également contre l'un des peptides structuraux de capside VP1, VP2 ou VP3.

9. Anticorps monoclonaux actifs à la fois contre les particules à antigénicité "C" et à antigénicité "D" d'un même poliovirus.

10. Les anticorps selon la revendication 9, caractérisés en ce qu'ils sont également actifs contre le peptide de structure de capside VP1.

11. Anticorps monoclonaux actifs à la fois contre un poliovirus à antigénicité "C" et l'un quelconque des peptides structuraux de capside VP1, VP2 ou VP3.

12. Anticorps selon l'une quelconque des revendications 8 à 11, caractérisés en ce qu'ils sont actifs contre des poliovirus ou peptides structuraux de poliovirus de type 1, notamment Mahoney.

13. Application des anticorps selon l'une quelconque des revendications 9 à 11 à l'identification de sous- unités peptidiques porteurs d'un déterminant antigénique commun aux poliovirus à antigénicité "C" et "D" et aux peptides de structure de capside VP1.

0112741

Office européen
des brevets

**RAPPORT DE RECHERCHE EUROPEENNE**

Numéro de la demande

EP  83 40 2230

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | CHEMICAL ABSTRACTS, vol. 97, no. 25, 20 décembre 1982, page 656, réf. no. 213907r, Columbus Ohio (US) & J. Virol. 1982, 43(3), 997-1005 E.A. EMINI et al.: "Poliovirus neutralization epitopes: analysis and localization with neutralizing monoclonal antibodies". * résumé * | 1-13 | C 12 N  15/00<br>C 12 N   5/00<br>C 12 P  21/00<br>G 01 N  33/54 |
| D,A | --- <br>VIROLOGY, vol. 115, no. 1, 1981 J. ICENOGLE et al.: "A neutralizing monoclonal antibody against poliovirus and its reaction with related antigens", pages 211-215. * en entier * | 1-13 | |
| A | ---<br>ABSTRACTS OF THE ANNUAL MEETING OF THE AMERICAN SOCIETY FOR MICROBIOLOGY, vol. 81, no. 0, 1981, résumé no. T 126, Bethesda, MD (US) F.K. KNAUERT et al.: "Monoclonal antibody specific for the D antigenic form of poliovirus", page 258. * résumé *<br><br>---        -/- | 1-13 | **DOMAINES TECHNIQUES RECHERCHES (Int. Cl. ³)**<br><br>C 12 N<br>C 12 P<br>G 01 N<br>A 61 K |

Le présent rapport de recherche a été établi pour toutes les revendications

| Lieu de la recherche<br>LA HAYE | Date d'achèvement de la recherche<br>23-02-1984 | Examinateur<br>RYCKEBOSCH A.O.A. |
|---|---|---|

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

EP  83 40 2230

| DOCUMENTS CONSIDERES COMME PERTINENTS | | | Page  2 | |
|---|---|---|---|---|
| Catégorie | Citation du document avec indication, en cas de besoin, des parties pertinentes | Revendication concernée | CLASSEMENT DE LA DEMANDE (Int. Cl. ³) | |
| D,A | C.R. ACAD. SC. PARIS, vol. 294, 11 janvier 1982, série III B. BLONDEL et al.: "Le polypeptide structural VP1 du poliovirus type I induit des anticorps neutralisants", pages 91-94. * en entier * | 1-13 | | |
| A | BIOLOGICAL ABSTRACTS, vol. 74, no. 5, 1982, résumé no. 36408, Philadelphia (US) A.D. OSTERHAUS et al.: "Monoclonal antibodies to polioviruses: production of specific monoclonal antibodies to the Sabin vaccine strains", page 3769 & INTERVIROLOGY 16(4): 218-224. 1981 * résumé * | 1-13 | | |
| P,X | BIOPASCAL, 83-X-0391421, 1983 (Informascience, Paris (FR)) B. BLONDEL et al.: "Detection by monoclonal antibodies of an antigenic determinant critical for poliovirus neutralization present on VP1 and on heat-inactivated virions" & Virology, vol. 126, no. 2, 707-710, 1983 * titre * | 1-13 | DOMAINES TECHNIQUES RECHERCHES (Int. Cl. ³) | |

Le présent rapport de recherche a été établi pour toutes les revendications

| Lieu de la recherche LA HAYE | Date d'achèvement de la recherche 23-02-1984 | Examinateur RYCKEBOSCH A.O.A. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03 82